# EUROPEAN PATENT APPLICATION

(11) **EP 0 920 864 A1**
(43) Date of publication of application: **09.06.1999**
(21) Application number: 98309273.5
(22) Date of filing: 12.11.1998
(51) Int. Cl.: A61K 31/44

(54) **Combination therapy including a specific beta-3 agonist and an anorectic agent**

(30) Priority: 03.12.1997 US 67268 P
(71) Applicant: Pfizer Products Inc., Groton, Connecticut 06340 (US)
(72) Inventor: Dow, Robert Lee, Waterford, Connecticut 06385 (US)
(74) Representative: Hayles, James Richard

(57) **Abstract**

This invention relates to pharmaceutical compositions and methods which have utility in treating obesity in animals and particularly in humans. The invention is particularly directed to such compositions which comprise a mixture of a compound which modifies eating behavior and (4-(2-(2-(6-aminopyridin-3-yl)-2(R)-hydroxyethylamino)ethoxy)phenyl)acetic acid, which is the compound of Formula I:

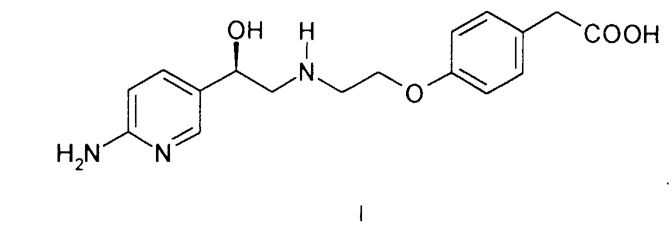

## Description

### BACKGROUND OF THE INVENTION

This invention relates to pharmaceutical compositions comprising a compound which modifies eating behavior and (4-(2-(2-(6-aminopyridin-3-yl)-2(R)-hydroxyethylamino)ethoxy)phenyl)acetic acid; or prodrugs thereof or pharmaceutically acceptable salts of said compounds or said prodrugs. These compositions have utility, *inter alia,* in the treatment of obesity in animals and in particularly in humans.

(4-(2-(2-(6-Aminopyridin-3-yl)-2(R)-hydroxyethylamino)ethoxy)phenyl)acetic acid has the structure of Formula I:

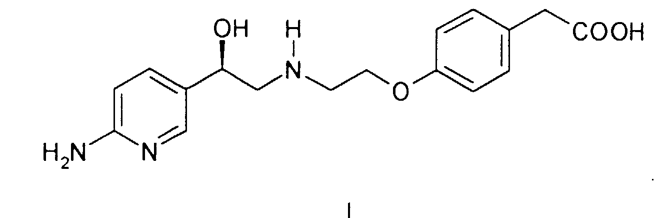

(4-(2-(2-(6-Aminopyridin-3-yl)-2(R)-hydroxyethylamino)ethoxy)acetic acid is disclosed in commonly assigned Intemational Patent Application Publication Number WO 96/35671, the disclosure of which is incorporated herein by reference, as a β-adrenergic agent. Accordingly, (4-(2-(2-(6-aminopyridin-3-yl)-2(R)-hydroxyethylamino)ethoxy)acetic acid has utility in the treatment of obesity.

β-Adrenergic agents have been categorized into β₁, β₂, and β₃ subtypes. Agonists of β-receptors promote the activation of adenyl cyclase. Activation of β₁ receptors invokes increases in heart rate. Activation of β₂ receptors induces relaxation of smooth muscle tissue which produces a drop in blood pressure and the onset of skeletal muscle tremors. Activation of β₃ receptors is known to stimulate lipolysis, which is the breakdown of adipose tissue triglycerides to glycerol and fatty acids. Activation of β₃ receptors also stimulates the metabolic rate, thereby increasing energy expenditure. Accordingly, activation of β₃ receptors promotes the loss of fat mass. Compounds that stimulate β receptors are therefore useful as anti-obesity agents.

International Patent Application WO 97/16189, the disclosure of which is incorporated herein by reference, discloses the use of selective β₃ receptor agonists in combination with compounds which modify eating behavior for the treatment of obesity.

### SUMMARY

This invention is directed to pharmaceutical compositions comprising a compound which modifies eating behavior, a prodrug thereof or a pharmaceutically acceptable salt of said compound or said prodrug; and (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug.

In one preferred aspect of this invention, said compound which modifies eating behavior is an anorectic agent.

In a more preferred aspect of this invention, said composition further comprises a pharmaceutically acceptable carrier or diluent.

In a still more preferred aspect of this invention, said anorectic agent is phenylpropanolamine, ephedrine, pseudoephedrine, phentermine, a Neuropeptide Y (hereinafter referred to as "NPY") antagonist, a cholecystokinin (hereinafter referred to as CCK-A) agonist, a monoamine reuptake inhibitor, a sympathomimetic agent, a serotoninergic agent, a dopamine agonist, a melanocyte-stimulating hormone receptor agonist or mimetic, a melanocyte-stimulating hormone analog, a cannabinoid receptor antagonist, a melanin concentrating hormone antagonist, the OB protein (hereinafter referred to as "leptin"), a leptin analog or a galanin antagonist.

In another preferred aspect of this invention, said anorectic agent is a bombesin agonist, dehydroepiandrosterone or analog thereof, glucocorticoid receptor agonist or analog thereof, an orexin receptor antagonist, a urocortin binding protein antagonist or a glucagon-like peptide-1 (insulinotropin) agonist.

In a still more preferred aspect of this invention, said monoamine reuptake inhibitor is sibutramine.

In another preferred aspect of this invention, said serotoninergic agent is dexfenfluramine or fenfluramine.

In yet another preferred aspect of this invention, said anorectic agent is leptin or a leptin analog.

In yet another preferred aspect of this invention, said dopamine agonist is bromocriptine.

In yet another preferred aspect of this invention, said anorectic agent is a CCK-A agonist.

In yet another preferred aspect of this invention, said anorectic agent is a galanin antagonist.

This invention is also directed to methods of treating obesity in an animal comprising administering to said animal a therapeutically effective amount of a compound which modifies eating behavior, a prodrug thereof or a pharmaceutically acceptable salt of said compound or said prodrug; and (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug.

In a preferred aspect of the obesity treating method of this invention, said compound which modifies eating behavior is an anorectic agent.

In a more preferred aspect of the obesity treating method, said anorectic agent is phenylpropanolamine, ephedrine, pseudoephedrine, phentermine, an NPY antagonist, a CCK-A agonist, a monoamine reuptake inhibitor, a sympathomimetic agent, a serotoninergic agent, a dopamine agonist, a melanocyte-stimulating hormone receptor agonist or mimetic, a melanocyte-stimulating hormone analog, a cannabinoid receptor antagonist, a melanin concentrating hormone antagonist, leptin, a leptin analog or a galanin antagonist.

In another preferred aspect of the obesity treating method, said anorectic agent is a bombesin agonist, dehydroepiandrosterone or analog thereof, glucocorticoid receptor agonist or analog thereof, an orexin receptor antagonist, a urocortin binding protein antagonist or a glucagon-like peptide-1 (insulinotropin) agonist.

In a still more preferred aspect of the obesity treating method, said monamine reuptake inhibitor is sibutramine.

In another preferred aspect of the obesity treating method, said serotoninergic agent is dexfenfluramine or fenfluramine.

In another preferred aspect of the obesity treating method, said anorectic agent is leptin or a leptin analog.

In another preferred aspect of the obesity treating method, said dopamine agonist is bromocriptine.

In another preferred aspect of the obesity treating method, said anorectic agent is a CCK-A agonist.

In another preferred aspect of the obesity treating method, said anorectic agent is a galanin antagonist.

This invention is also directed to methods of treating diabetes in an animal comprising administering to said animal a therapeutically effective amount of a compound which modifies eating behavior, a prodrug thereof or a pharmaceutically acceptable salt of said compound or said prodrug; and (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug.

A preferred aspect of the diabetes treating method of of this invention is wherein said compound which modifies eating behavior is an anorectic agent.

A still more preferred aspect of the diabetes treating method of this invention is wherein said anorectic agent is phenylpropanolamine, ephedrine, pseudoephedrine, phentermine, an NPY antagonist, a CCK-A agonist, a monoamine reuptake inhibitor, a sympathomimetic agent, a serotoninergic agent, a dopamine agonist, a melanocyte-stimulating hormone receptor agonist or mimetic, a cannabinoid receptor antagonist, a melanocyte- stimulating hormone analog, a melanin concentrating hormone antagonist, leptin, a leptin analog or a galanin antagonist.

Another preferred aspect of the diabetes treating method of this invention is wherein said anorectic agent is a bombesin agonist, dehydroepiandrosterone or analog thereof, glucocorticoid receptor agonist or analog thereof, an orexin receptor antagonist, a urocortin binding protein antagonist or a glucagon-like peptide-1 (insulinotropin) agonist.

A still more preferred aspect of the diabetes treating method of of this invention is wherein said monamine reuptake inhibitor is sibutramine.

Another preferred aspect of the diabetes treating method of of this invention is wherein said serotoninergic agent is dexfenfluramine.

Yet another preferred aspect of the diabetes treating method of this invention is wherein said anorectic agent is leptin or a leptin analog.

Yet another preferred aspect of the diabetes treating method of this invention is wherein said dopamine agonist is bromocriptine.

Yet another preferred aspect of the diabetes treating method of this invention is wherein said anorectic agent is a CCK-A agonist.

Yet another preferred aspect of the diabetes treating method of this invention is wherein said anorectic agent is a galanin antagonist.

This invention is also directed to a kit comprising:
a. an amount of a compound which modifies eating behavior, a prodrug thereof or a pharmaceutically acceptable salt of said compound or said prodrug in a first unit dosage form;
b. an amount of (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug in a second unit dosage form; and
c. a container.

In another aspect of this invention, the first unit dosage form of said kit further comprises a pharmaceutically acceptable carrier or diluent.

A preferred aspect of this invention comprises an anorectic agent.

In another aspect of this invention, the second unit dosage form of said kit further comprises a pharmaceutically acceptable carrier or diluent.

A preferred aspect of this invention comprises an anorectic agent.

In another aspect of this invention, both the first and second unit dosage forms further comprise a pharmaceutically acceptable carrier or diluent.

A preferred aspect of this invention comprises an anorectic agent.

### DETAILED DESCRIPTION OF THE INVENTION

The compound of Formula I which is used in the method and pharmaceutical compositions of the instant invention is readily prepared according to methods well known to those skilled in the art particularly as described in International Patent Application Publication No. WO 96/35671. Specifically, the compound of Formula I is prepared by reacting a carboxylic acid ester derivative or other protected carboxylic acid derivative of the compound of Formula I with a base such as potassium hydroxide in an aqueous solvent such as ethanol/water, methanol/water or the like. The reaction mixture is stirred at a temperature of about 0°C to about 25°C until the reaction is complete as determined by thin layer chromatography or other analytical technique. Generally the reaction mixture is stirred for four hours. The product is generally isolated by adjusting the pH of the reaction mixture to the isoelectric point of the compound of Formula I and filtering to obtain the solid product. When the carboxylic acid ester derivative used in this method is racemic, the product is the racemate of the compound of Formula I.

The compound of Formula I which is used in the methods and compositions of the instant invention is an optically active compound and is designated the 2(R) enantiomer. This enantiomer is prepared from the racemate according to resolution methods well known to those skilled in the art.

Alternatively, the compound of Formula I may be prepared from optically active intermediates as set forth below.

The compound of Formula I can be synthesized from compounds of Formula II 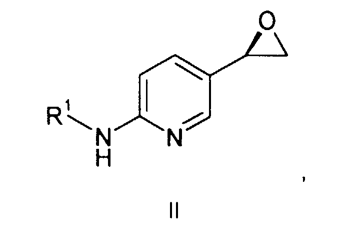 wherein R¹ is a suitable amine protecting group, by a coupling reaction with an amine of Formula III 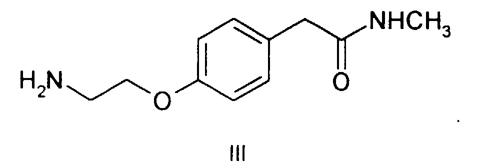 This amine is prepared according to methods well known to those skilled in the art or by the procedures set forth in Preparation Nine below. This coupling reaction is typically carried out by reacting said amine with an epoxide of Formula II in a polar aprotic solvent such as dimethyl sulfoxide, dimethyl formamide, acetonitrile or a lower alkanol such as ethanol, 2-propanol or butanol at a temperature from about - 10°C to about 125°C. Preferably the solvent is dimethyl sulfoxide and the reaction is carried out at a temperature from about 0°C to about 80°C.

The compounds of Formula II may be prepared by treating a compound of Formula IV 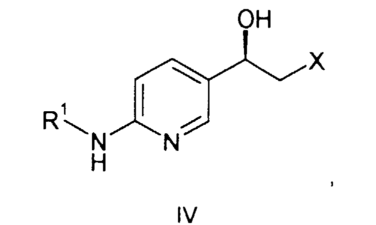 wherein R¹ is a suitable amine protecting group and X is a suitable leaving group such as halo or organosulfonyloxy, with a non-nucleophilic base. Generally, it is preferred that the non-nucleophilic base be selected from the group consisting of sodium hydroxide, potassium hydroxide, sodium hydride, potassium *tert*-butoxide or 1,8-diazabicyclo[5.4.0]undec-7-ene. The reaction is preferably conducted by stirring the substrate compound of Formula IV together with the appropriate non-nucleophilic base in a reaction inert solvent at a temperature of about -20 °C to about 100 °C. Where used herein, the term reaction inert solvent refers to any solvent or solvent system which does not interact with starting materials, reagents, intermediates or products in a manner which adversely affects the reaction or the yield of the desired product. With respect to this particular reaction, it is preferred that the solvent is a polar, non-hydroxylic solvent such as an ether derivative including but not limited to tetrahydrofuran, dioxane and dimethoxyethane; chlorinated hydrocarbons including but not limited to carbon tetrachloride, chloroform and methylene chloride; aromatic hydrocarbons including but not limited to benzene, toluene and xylene; dimethylformamide; dimethylsulfoxide or any mixture of these solvents. Generally the most preferred solvent is tetrahydrofuran.

When the compounds of Formula IV disclosed herein are organosulfonyloxy derivatives, said compounds may be prepared by reacting an appropriate compound of Formula V 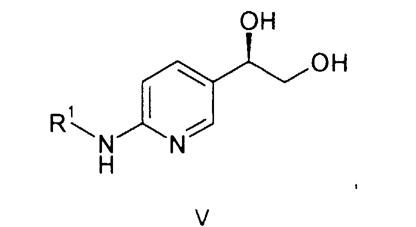 wherein R¹ is a suitable amine protecting group, with an organosulfonyl chloride in the presence of a suitable base. Suitable bases which may be used to effect this transformation include the lower trialkylamines, pyridine and pyridine derivatives. Preferred bases within those groups include but are not limited to triethylamine, diisopropylethylamine, 2,4,6-collidine and 2,6-lutidine. Pyridine is the most preferred base. Suitable organosulfonyl chlorides include methanesulfonyl chloride, p-nitrobenzenesulfonyl chloride, m-nitrobenzenesulfonyl chloride, p-toluenesulfonyl chloride and benzenesulfonyl chloride. A generally preferred organosulfonyl chloride derivative is p-toluenesulfonyl chloride. The reaction is conveniently conducted by stirring the desired substrate compound of Formula V together with the appropriate organosulfonyl chloride in a reaction inert solvent at a temperature of about -20 °C to about 50 °C. It is preferred that the solvent is a polar solvent such as an ether derivative including but not limited to tetrahydrofuran, dioxane and dimethoxyethane; chlorinated hydrocarbons including but not limited to carbon tetrachloride, chloroform and methylene chloride; aromatic hydrocarbons including but not limited to benzene, toluene and xylene; dimethylformamide; N-methyl-2-pyrrolidinone; dimethylacetamide; pyridine or any mixture of these solvents. Generally the most preferred solvent is pyridine. Due to the presence of chloride ion in this reaction, the reaction product may be contaminated with 2-chloro derivatives. These mixtures can be converted entirely to the 2-chloro derivatives as described below.

To prepare the compounds of Formula IV wherein X is halo, the 2-organosulfonyloxy derivatives of the compound of Formula IV or mixtures thereof containing 2-chloro derivatives of the compound of Formula IV are reacted with a halogenating agent in a reaction inert solvent. The reaction may be conducted conveniently at a temperature of from about 25°C to the reflux temperature of the solvent utilized. It is generally preferred to conduct the reaction at the reflux temperature. Halogenating agents are compounds which are capable of transferring a halo group to an organic substrate, said substrate having a leaving group which can be displaced by said halide ion. Preferred halogenating agents are lithium halides. A particularly preferred chlorinating agent used to prepare the compounds of Formula IV wherein X is chloro is lithium chloride. A preferred solvent is ethanol.

The compounds of Formula V disclosed herein may be prepared by reacting an appropriate compound of Formula VI 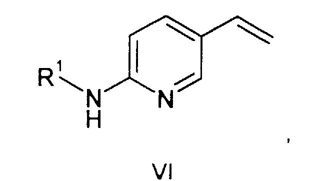 wherein R¹ is a suitable amine protecting group, with a catalyst comprised of osmium (VIII) oxide or an osmium salt, in the presence of an auxiliary oxidizing agent, and optionally in the presence of a chiral auxiliary ligand such as (DHQD)₂PHAL or (DHQD)₂PYR and an auxiliary base. When it is desirable to use a catalyst other than osmium (VIII) oxide in this reaction, the catalyst is generally selected from osmium metal, potassium osmate (Vl) dihydrate and osmium (III) chloride. Generally, it is preferred to use osmium tetroxide as the catalyst when conducting this reaction. Auxiliary oxidizing agents that may be employed include but are not limited to potassium ferricyanide, sodium ferricyanide, potassium persulfate, sodium persulfate, potassium chlorate, sodium chlorate and N-methylmorpholine-N-oxide (the latter oxidizing agent may only be used in the absence of chiral auxiliary ligands such as (DHQD)₂PHAL or (DHQD)₂PYR). It may also be desirable to use a mixture of auxiliary oxidizing agents to achieve optimum performance in this reaction. An especially suitable mixture of auxiliary oxidizing agents is sodium persulfate and potassium ferricyanide. Chiral auxiliary ligands that may be used, in addition to those already recited, include hydroquinidine indolinediyl diether ((DHQD)IND), hydroquinine phthalazinediyl diether ((DHQ)₂PHAL), hydroquinine pyrimindinediyl diether ((DHQ)₂PYR), hydroquinine indolinediyl diether ((DHQ)IND), hydroquinidine phenanthrinediyl diether (DHQD-PHN) and hydroquinine phenanthrinediyl diether (DHQ-PHN). The reaction is typically conducted by stirring the desired substrate compound of Formula VI together with the appropriate reagents recited above in a polar solvent at a temperature of about -10°C to about 70°C. The reaction is conveniently conducted at about 20°C. Polar solvents which are generally useful in this reaction include water, a lower alkanol, an ether or a mixture of any of these solvents. A lower alkanol is an alcohol containing from one to four carbon atoms.

The dihydroxylation reaction disclosed in the preceding paragraph may be conducted either in the presence or in the absence of a chiral auxiliary ligand. When the reaction is conducted in the absence of a chiral auxiliary ligand, the diol product is racemic. When the reaction is conducted in the presence of a chiral auxiliary ligand, the dihydroxylation reaction proceeds stereoselectively, resulting in an essentially optically pure diol product.

The compounds of Formula VI disclosed herein may be prepared by reacting a compound of Formula VII 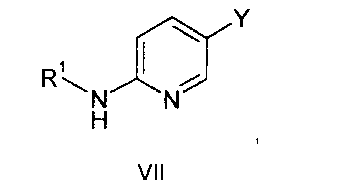 wherein R¹ is a suitable amine protecting group and Y is halo, with ethylene gas in the presence of a base, a phosphine derivative and a palladium catalyst. Suitable bases for the reaction include lower trialkylamines, sodium carbonate, potassium carbonate, sodium bicarbonate and potassium bicarbonate. Generally, triethylamine is preferred. Suitable phosphine derivatives include triarylphosphines such as triphenylphosphine, diphenyl-2-pyridylphosphine and tri-o*rtho*-tolylphosphine, with the latter being generally preferred. When Y is iodo, the palladium catalyst may be selected from a variety of palladium salts and complexes such as but not limited to palladium metal on carbon or some other suitable solid support, allylpalladium chloride dimer, palladium (II) chloride, palladium (II) acetate, palladium (0) tetrakis(triphenylphosphine), palladium (II) bis(triphenylphosphine) chloride, palladium (0) bis(dibenzylideneacetone) and palladium (0) bis(benzonitrile). When Y is bromo or trifluoromethanesulfonyloxy, the palladium catalyst may be selected from a variety of palladium salts and complexes such as but not limited to allylpalladium chloride dimer, palladium (II) chloride, palladium (II) acetate, palladium (0) tetrakis(triphenylphosphine), palladium (II) bis(triphenylphosphine) chloride, palladium (0) bis(dibenzylideneacetone), palladium (0) bis(benzonitrile and allylpalladium chloride dimer. Palladium (II) acetate is especially preferred. The reaction is typically conducted by stirring the compound of Formula VII together with the above recited reagents in a polar solvent at a temperature of about 20 °C to about 150 °C under an atmosphere of ethylene at a pressure of about 1 atmosphere to about 10 atmospheres. The preferred polar solvents for use in this reaction include, but are not limited to ethers, such as tetrahydrofuran, dimethoxyethane and dioxane; lower trialkylamines, such as triethylamine, diisopropylethylamine and tributylamine; aromatic hydrocarbons, such as benzene, toluene and xylene; dimethylformamide; N-methyl-2-pyrrolidone; acetonitrile; dimethylacetamide; or a mixture of any of these solvents. Acetonitrile is an especially preferred solvent.

The compounds of Formula III above are prepared according to procedures well known to those skilled in the art or in a manner analogous to the procedures set forth in Preparations Six to Nine below.

The compounds of Formula VII above are prepared from the commercially or readily available 2-amino-3-bromo-pyridine according to procedures well known to those skilled in the art or in a manner analogous to that set forth in Preparation One.

If not commercially available, the necessary starting materials for the chemical reactions disclosed herein may be prepared by procedures which may be selected from standard organic chemical techniques found in standard organic textbook references. The techniques found therein may be applied directly to the synthesis of known starting materials described directly in that reference or may be applied by analogy to compounds having similar functionality to achieve predictable results.

The second compound of the combination of this invention is an eating behavior modifying compound. Compounds which modify eating behavior include anorectic agents, which are compounds which diminish the appetite.

Anorectic agents within the scope of this invention include phenylpropanolamine, ephedrine, pseudoephedrine, sympathomimetic agents, NPY antagonists, CCK-A agonists, monoamine reuptake inhibitors, serotoninergic agents, dopamine agonists, melanocyte-stimulating homone receptor agonists or mimetics, cannabinoid receptor antagonists, melanocyte-stimulating hormone analogs, melanin concentrating hormone antagonists, leptin, leptin analogs or galanin antagonists.

A particularly preferred monoamine reuptake inhibitor is sibutramine, which can be prepared as disdosed in U.S. 4,929,629, the disclosure of which is incorporated herein by reference.

Preferred serotoninergic agents include fenfluramine and dexfenfluramine, which can be prepared as disclosed in U.S. 3,198,834, the disclosure of which is incorporated herein by reference.

A particularly preferred dopamine agonist is bromocriptine, which can be prepared as disclosed in U.S. 3,752,814 and U.S. 3,752,888, the disclosures of which are incorporated herein by reference.

Another preferred anorectic agent is phentermine, which can be prepared as disclosed in U.S. 2,408,345, the disclosure of which is incorporated herein by reference.

It will be appreciated by those skilled in the art that the eating behavior modifying compounds used in the combinations and methods of this invention may contain at least one chiral center. Accordingly, those compounds may exist in, and be isolated in, optically active and racemic forms. Some compounds may exhibit polymorphism. It is to be understood that the present invention encompasses any racemic, optically active, polymorphic or stereoisomeric form, or any mixture thereof, which form possesses properties useful in the treatment of obesity or useful as intermediates in the preparation of any compounds useful in the treatment of obesity, it being well known in the art how to prepare optically active forms (for example, by resolution of the racemic form by recrystallization techniques, by synthesis from optically active starting materials, by chiral synthesis or by chromatographic separation using a chiral stationary phase) and how to determine efficacy for the treatment of obesity.

Conventional methods and techniques of purification and separation known to those skilled in the art may be used to isolate the compound of Formula I, prodrugs thereof and pharmaceuctically acceptable salts of said compound or said prodrugs of this invention and any of the eating behavior modifying compounds, prodrugs thereof and pharmaceutically acceptable salts of said compounds and said prodrugs used in the methods or combinations of this invention. Such techniques include all types of chromatography, including but not limited to high performance liquid chromatography, column chromatography using common adsorbents such as silica gel, thin layer chromatography and the like; recrystallization; and differential (i.e., liquid-liquid) extraction techniques.

As used in the specification and appendant claims, the following terms have the meanings described. The terms alkyl and alkoxy include both straight and branched chain radicals, but it is to be understood that references to individual radicals such as propyl or propoxy embrace only the straight chain radical unless reference is specifically made to for example isopropyl or isopropoxy, in which case the branched chain isomer is meant.

The term halo, unless otherwise indicated, indudes chloro, flouro, bromo and iodo.

The terms "Ad-mix-A" and "Ad-mix-α" are synonymous names for a reagent used in this invention and sold by Aldrich Chemical Co. The reagent contains the chiral ligand (DHQ)₂PHAL, the catalyst potassium osmate (VI) dihydrate, the auxiliary oxidizing agent potassium ferricyanide and the base potassium carbonate. The reagent is used in the asymmetric dihydroxylation of olefins. The reagent is sold by Aldrich under a license from Sepracor, Inc. of Marlborough, Massachusetts. (see Aldrich catalog, 1996-97, page 444.)

The terms "Ad-mix-B" and "Ad-mix-β" are synonymous names for a reagent used in this invention and sold by Aldrich Chemical Co. The reagent contains the chiral ligand (DHQD)₂PHAL, the catalyst potassium osmate (VI) dihydrate, the auxiliary oxidizing agent potassium ferricyanide and the base potassium carbonate. The reagent is used in the asymmetric dihydroxylation of olefins. The reagent is sold by Aldrich under a license from Sepracor, Inc. of Marlborough, Massachusetts. (see Aldrich catalog, 1996-97, page 444.)

The term "suitable amine protecting group" defines an organic radical which is readily attached to an amine nitrogen atom of a substrate and which blocks said nitrogen atom from reacting with reagents and substrates used in and intermediates and transition state molecules formed in subsequent chemical transformations. Said organic radical is readily removable under mild conditions. Where used herein, the phrase "mild conditions" defines conditions which are capable of removing a protecting group but which do not have any effect upon any other portions of the substrate to which said protecting group is attached. Preferred amine protecting groups include (C₁-C₆)alkyl carbonyl, (C₁-C₆) alkoxycarbonyl and (C₁-C₆) alkoxysulfonyl. A particularly preferred amine protecting group is acetyl.

The term "suitable leaving group" includes a group which may be readily displaced by a nucleophile which has a greater affinity for the positively charged carbon atom to which said leaving group is attached than said leaving group. Preferred leaving groups are halo and organosulfonyloxy groups. A preferred halo group is chloro. Particularly preferred leaving groups are organosulfonyloxy groups. Particularly preferred organosulfonyloxy groups are methanesulfonyloxy, trifluoromethanesulfonyloxy, benzenesulfonyloxy, p-toluenesulfonyloxy, p-nitrobenzenesulfonyloxy or m-nitrobenzenesulfonyloxy.

The term "suitable base" includes a base which, when added to the reaction mixture in which said base is to operate, increases the pH of the reaction mixture or operates on the substrate to remove a proton from said substrate or otherwise render said substrate susceptible to electrophilic attack without affecting other potentially reactive functional groups in said substrate.

The eating behavior modifying compounds used in the methods and combinations of the instant invention may contain basic moieties which are suitable for reaction with an appropriate acid to form a pharmaceutically acceptable acid addition salt of said eating behavior modifying compound. The expression "pharmaceutically-acceptable acid addition salts" is intended to include but not be limited to such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts.

The acid addition salts of the compound of Formula I and the eating behavior modifying compounds are readily prepared by reacting the base forms of those compounds with an appropriate acid. When the salt is of a monobasic acid (e.g., the hydrochloride, the hydrobromide, the p-toluenesulfonate, the acetate) the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However, when such salts as the sulfate, the hemisuccinate, the hydrogen phosphate or the phosphate are desired, the appropriate and exact chemical equivalents of acid will generally be used. The free base and the acid are conveniently combined in a co-solvent from which the desired salt precipitates or can otherwise be isolated by concentration and addition of a non-solvent or by simple addition of a non-solvent without concentration or by lyophilization of an aqueous solution of said salt.

The eating behavior modifying compounds used in the methods and combinations of the instant invention may contain acidic moieties which are suitable for reaction with an appropriate base to form a pharmaceutically acceptable cationic salt of said eating behavior modifying compound. The compound of Formula I forms pharmaceutically acceptable cationic salts by reacting the free carboxylic acid with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine and tromethamine. The salt is isolated by concentration to dryness or by addition of a non-solvent. In many cases, a salt is preferably prepared by mixing a solution of the acid with a solution of a different salt of the cation (sodium or potassium ethylhexanoate, magnesium oleate), employing a solvent (e.g., ethyl acetate) from which the desired cationic salt precipitates, or can be otherwise isolated by concentration and/or addition of a non-solvent.

The expression "prodrug" refers to compounds that are drug precursors which, following administration, release the drug *in vivo* via some chemical or physiological process (e.g., a prodrug on being brought to the physiological pH or through enzyme action is converted to the desired drug form). Exemplary prodrugs upon cleavage release the corresponding free acid, and such hydrolyzable ester-forming residues of the compound of Formula I and eating behavior modifying compounds include but are not limited to those compounds wherein the free hydrogen of a carboxyl group is replaced by (C₁-C₄)alkyl, (C₂-C₇)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as b-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl. The prodrugs within the scope of this invention are prepared using methods well known to those skilled in the art.

The amino acid prodrugs of this invention may be prepared by conventional peptide coupling reactions coupling a free amino or carboxylic group of the compound of formula I with an amino acid or a polypeptide, e.g. dipeptide chain. The coupling reaction is generally conducted at a temperature of about -30° to about 80° C., preferably about 0° to about 25°C. Suitable coupling reagents are usually present, such as dicyclohexylcarbodiimide with hydroxybenzotriazole (HBT), N-3-dimethylaminopropyl-N'-ethylcarbodiimide with HBT, 2-ethoxy-1-ethoxycarbonyl-1,2-dihydroquinoline, carbonyl diimidazole with HBT, or diethylphosphoryl-cyanide. The reaction is generally conducted in an inert solvent such as acetonitrile, methylene chloride, chloroform, dimethylformamide, dioxane, tetrahydrofuran, dimethoxyethane, or water, or a mixture of two or more such solvents.

Ester, carbonate or carbamate prodrugs of this invention may be prepared by reaction of the free hydroxyl or amino group of the compound of formula I with an activated carbonyl containing molecule such as acetyl chloride or ethyl chloroformate. The reaction can be carried out neat or in the presence of a reaction inert solvent such as methylene chloride, at a temperature from about -78°C to about 100°C. The alcohol moiety of Formula I can also be reacted with cyanogen chloride in the presence of a Lewis acid to form carbamates.

When treating obesity, generally satisfactory results are obtained when the combination of the instant invention, i.e., an eating behavior modifying compound in combination with the compound of Formula I, prodrugs, or pharmaceutically acceptable salts thereof (hereinafter also referred to herein as "active ingredients or compounds") are administered to animals, including humans, *via* either the oral or the parenteral route. Administration by the oral route is preferred, being more convenient and avoiding the possible pain and irritation of injection. However, in circumstances where the subject cannot swallow the medication, or absorption following oral administration is impaired, as by disease or other abnormality, it is essential that the drug be administered parenterally. By either route, the dosage of the compound of Formula I is the range of about 0.01 to about 50 mg/kg/day body weight of the subject per day, preferably about 0.3 to about 30 mg/kg/day body weight per day, and most preferably about 1 to about 10 mg/kg/day body weight, administered singly or as a divided dose. The dosage of the compound which modifies eating behavior is in the range of about 0.01 to about 50 mg/kg/day body weight, preferably about 0.1 mg/kg/day to about 10 mg/kg/day body weight, administered singly or as a divided dose. Particularly, when the compound which modifies eating behavior is (1) sibutramine, the dosage of sibutramine is about 0.01 mg/kg/day to about 30 mg/kg/day body weight, preferably about 0.1 mg/kg/day to about 1 mg/kg/day body weight; (2) dexfenfluramine, the dosage of dexfenfluramine is about 0.01 mg/kg/day to about 30 mg/kg/day body weight, preferably about 0.1 mg/kg/day to about 1 mg/kg/day body weight; (3) bromocriptine, the dosage of bromocriptine is about 0.01 to about 10 mg/kg/day body weight, preferably 0.1 mg/kg/day to about 10 mg/kg/day body weight; (4) phentermine, the dosage of phentermine is about 0.01 mg/kg/day to about 10 mg/kg/day, preferably about 0.1 mg/kg/day to about 1 mg/kg/day body weight. However, the optimum dosage for the individual subject being treated will be determined by the person responsible for the treatment, generally smaller doses being administered initially and thereafter increments made to determine the most suitable dosage. This will vary according to the particular compound employed and with the subject being treated.

The combinations of the present invention are generally used together with a pharmaceutically acceptable carrier or diluent. Suitable pharmaceutically-acceptable carriers include inert solid fillers or diluents and sterile aqueous or organic solutions. The active compounds will be present in such pharmaceutical compositions in amounts sufficient to provide the desired dosage amount in the range described above. Thus, for oral administration the compounds can be combined with a suitable solid or liquid carrier or diluent to form capsules, tablets, powders, syrups, solutions, suspensions and the like. The pharmaceutical compositions may, if desired, contain additional components such as flavorants, sweeteners, excipients and the like.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

These compositions may also be administered parenterally. For parenteral administration the compositions can be combined with sterile aqueous or organic media to form injectable solutions or suspensions. Solutions or suspensions of these compositions can be prepared in water suitably mixed with a surfactant such as hydroxypropylcellulose. Dispersions can also be prepared in sesame or peanut oil, ethanol, water, polyol (e.g., glycerol, propylene glycol and liquid polyethylene glycol), suitable mixtures thereof, vegetable oils, N-methyl glucamine, polyvinylpyrrolidone and mixtures thereof in oils as well as aqueous solutions of water-soluble pharmaceutically acceptable salts of the compounds. Under ordinary conditions of storage and use, these preparations contain a preservative to prevent the growth of microorganisms. The injectable solutions prepared in this manner can then be administered intravenously, intraperitoneally, subcutaneously, or intramuscularly, with intramuscular administration being the preferred parenteral route in humans.

The pharmaceutical forms suitable for injectable use include sterile aqueous solutions or dispersions and sterile powders for the preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi.

The effective dosage of the active ingredients employed may vary depending on the particular compound employed, the mode of administration and the severity of the obesity being treated.

As a consequence of their action in reducing body fat (lipolysis) the compositions of the present invention possess utility for treating obese household pets, for example companion animals such as dogs and cats. The administration of a composition of this invention can be effected orally or non-orally, for example by injection. An amount of a composition of this invention is administered such that an effective dose is received, generally a daily dose. When administered orally to an animal, the composition of this invention contains a compound of Formula I, prodrug thereof or a pharmaceutically acceptable salt of said compound or said prodrug, dosed at a range between 0.01 and 50 mg/kg/day of body weight, preferably between 0.3 and 30 mg/kg/day of body weight and most preferably between 0.1 mg/kg/day and 10 mg/kg/day body weight. The second compound of this invention, when adminstered to an animal, is dosed at a range between about 0.01 to about 50 mg/kg/day body weight, preferably about 0.1 mg/kg/day to about 10 mg/kg/day body weight, administered singly or as a divided dose. Particularly, when the second compound of this invention is (1) sibutramine, the dosage of sibutramine is about 0.01 mg/kg/day to about 30 mg/kg/day body weight, preferably about 0.1 mg/kg/day to about 1 mg/kg/day body weight; (2) dexfenfluramine, the dosage of dexfenfluramine is about 0.01 mg/kg/day to about 30 mg/kg/day body weight, preferably about 0.1 mg/kg/day to about 1 mg/kg/day body weight; (3) bromocriptine, the dosage of bromocriptine is about 0.01 to about 10 mg/kg/day body weight, preferably 0.1 mg/kg/day to about 10 mg/kg/day body weight; (4) phentermine, the dosage of phentermine is about 0.01 mg/kg/day to about 10 mg/kg/day, preferably about 0.1 mg/kg/day to about 1 mg/kg/day body. Conveniently, the medication can be carried in the drinking water so that a therapeutic dosage of the agent is ingested with the daily water supply. The agent can be directly metered into drinking water, preferably in the form of a liquid, water-soluble concentrate (such as an aqueous solution of a water soluble salt).

Conveniently, the active ingredients can also be added directly to the companion animal's feed, as such, or in the form of an animal feed supplement, also referred to as a premix or concentrate. A premix or concentrate of therapeutic agent in a carrier is more commonly employed for the inclusion of the agent in the feed. Suitable carriers are liquid or solid, as desired, such as water, various meals such as alfalfa meal, soybean meal, cottonseed oil meal, linseed oil meal, corncob meal and corn meal, molasses, urea, bone meal, and mineral mixes. A particularly effective carrier is the respective animal feed itself; that is, a small portion of such feed. The carrier facilitates uniform distribution of the active materials in the finished feed with which the premix is blended. It is important that the compound be thoroughly blended into the premix and, subsequently, the feed. In this respect, the agent may be dispersed or dissolved in a suitable oily vehicle such as soybean oil, corn oil, cottonseed oil, and the like, or in a volatile organic solvent and then blended with the carrier. It will be appreciated that the proportions of active material in the concentrate are capable of wide variation since the amount of agent in the finished feed may be adjusted by blending the appropriate proportion of premix with the feed to obtain a desired level of therapeutic agent.

High potency concentrates may be blended by the feed manufacturer with proteinaceous carrier such as soybean oil meal and other meals, as described above, to produce concentrated supplements which are suitable for direct feeding to animals. In such instances, the animals are permitted to consume the usual diet. Alternatively, such concentrated supplements may be added directly to the feed to produce a nutritionally balanced, finished feed containing a therapeutically effective level of a compound according to the invention. The mixtures are thoroughly blended by standard procedures, such as in a twin shell blender, to ensure homogeneity.

If the supplement is used as a top dressing for the feed, it likewise helps to ensure uniformity of distribution of the active material across the top of the dressed feed.

The preferred domestic pet feeds usually contain about 1 to 400 grams and preferably 10 to 400 grams of active ingredient per ton of feed.

In general, parenteral administration involves injection of a sufficient amount of the compounds of the present invention to provide the animal with about 0.01 to 50 mg/kg/day of body weight of the compound of Formula I and about 0.01 to 30 mg/kg/day of a compound which modifies eating behavior. The preferred dosage for domestic pets is in the range of from 0.3 to 30 mg/kg/day of body weight of the compound of Formula I and about 0.1 to 10 mg/kg/day of a compound which modifies eating behavior.

Paste formulations can be prepared by dispersing the active compound in a pharmaceutically acceptable oil such as peanut oil, sesame oil, corn oil or the like.

Pellets containing an effective amount of the compound of the present invention can be prepared by admixing the compound of the present invention with a diluent such as carbowax, carnuba wax, and the like, and a lubricant, such as magnesium or calcium stearate, can be added to improve the pelleting process.

The present invention has several advantagous veterinary feature, e.g., for the pet owner or veterinarian who wishes to increase leanness and trim unwanted fat from pet or companion animals, the present invention provides the means by which this can be accomplished.

Since the present invention has an aspect that relates to a combination of active ingredients which may be administered separately, the invention also relates to combining separate pharmaceutical compositions in kit form. The kit comprises two separate pharmaceutical compositions: the compound of Formula I, a prodrug thereof or a pharmaceutically acceptable salt of said compound or said prodrug and a compound which modifies eating behavior, a prodrug thereof or a pharmaceutically acceptable salt of said prodrug or said compound. The kit also comprises a container. The container is used to contain the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card, e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also, a daily dose of the Formula I compound, a prodrug thereof or a pharmaceutically acceptable salt of said compound or said prodrug can consist of one tablet or capsule while a daily dose of the second compound, a compound which modifies eating behavior, a prodrug thereof or a pharmaceutically acceptable salt of said prodrug or said compound, can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered microchip memory coupled with a liquid crystal readout or audible reminder signal which, for example, reads out the date that the last daily dose has been taken and/or reminds one when the next dose is to be taken.

Methods of preparing various pharmaceutical compositions with a certain amount of active ingredient are known, or will be apparent in light of this disclosure, to those skilled in this art. For examples, see Remington's Pharmaceutical Sciences, Mack Publishing Company, Easter, Pa., 15th Edition (1975).

Pharmaceutical compositions according to the invention may contain 0.1%-95% of the compound(s) of this invention, preferably 1%-70%. In any event, the composition or formulation to be administered will contain a quantity of a compound(s) according to the invention in an amount effective to treat the disease/condition of the subject being treated, i.e., obesity.

The compound of Formula I used in the compositions of the present invention is prepared as set forth in Examples One and Two.

### Example One

### (4-(2-(2-(6-Aminopyridin-3-yl)-2(R)-hydroxyethylamino)ethoxy)acetic acid

Step A: Methyl (4-(2-t-butoxycarbonylaminoethoxy)phenyl)acetate. To a stirred solution of methyl 4-hydroxphenyl acetate (4.00 g, 24.1 mmol) and triphenylphosphine (9.50 g, 36.1 mmol) in THF (24 mL) were added solutions of 2-(t-butoxycarbonylamino)ethanol (5.80 g, 36.1 mmol) and diethylazodicarboxylate (5.70 mL, 36.1 mmol) in THF (6 mL, each) simultaneously over a 1.5 h period. After an additional 3 h, the reaction was concentrated *in vacuo* and subjected to flash chromatography (600 g silica gel, 20% ethyl acetate/hexanes) to afford a golden oil, 5.78 g. ¹H NMR (CDCl₃) δ 7.15 (d, 2H), 6.80 (d, 2H), 4.95 (br s, 1H), 3.98 (m, 2H), 3.65 (s, 3H), 3.54 (s, 2H, 3.48 (m, 2H) 1.44 (s, 9H).
Step B: Methyl(4-(2-aminoethoxy)phenyl)acetate. To a cooled (5°C), stirred solution of methyl (4-(2-t-butoxycarbonylaminc-ethoxy)phenyl) acetate (prepared as described in Example One, Step A, 5.75 g, 18.6 mmol) in dichloromethane (20 mL) was added trifluoroacetic acid (6 mL). The resulting solution was stirred at ambient temperature for 2 h, diluted in ethyl acetate, washed with half-saturated sodium carbonate, brine, dried (Na₂SO₄) and concentrated *in vacuo* to afford an orange oil, 3.25 g. ¹H NMR (CDCl₃) δ 7.10 (d, 2H), 6.77 (d, 2H), 3.92 (t, 2H), 3.60 (s, 3H), 2.49 (s, 2H), 3.00 (t, 2H).
Step C: Methyl(4-(2-(2(R)-hydroxy-2-tetrazolo[1,5-a]pyridin-6-ylethylamino)ethoxy)-Phenyl)acetate. A solution of methyl (4-(2-aminoethoxy)phenyl) acetate (prepared as described in Example One, Step B, 0.56 g, 2.71 mmol) and (2R)-(tetrazolo[1,5-a]pyrid-6-yl)oxirane (0.40 g, 2.47 mmol), generated as in Example 1 of U.S. 5,030,640, in methanol (7.5 mL) was heated at reflux temperature for 7 h. Concentration of the reaction solution *in vacuo* afforded a solid which was subjected to flash chromatography (3% methanol/chloroform) to afford the title compound of Step C as a colorless solid, 0.52 g. ¹H NMR (CDCl₃) δ 8.86 (s, 1H), 7.92 (d, 1H), 7.57 (d, 1H), 7.15 (d, 2H), 6.81 (d, 2H), 4.83 (dd, 1H), 4.06 (t, 2H), 3.66 (s, 3H), 3.56 (s, 2H), 3.20-3.02 (m, 3H), 2.76 (dd, 1H).
Step D: Methyl (4-(2-(2-(6-aminopyridin-3-yl)-2(R)-hydroxyethylamino)ethoxy)phenyl)acetate. A slurry of methyl (4-(2-(2(R)-hydroxy-2-tetrazolo[1,5-a]pyridin-6-ylethylamino)-ethoxy)phenyl) acetate (prepared as described in Example One, Step C, 0.51 g, 1.37 mmol) and stannous chloride-dihydrate (0.93 g, 4.12 mmol) in methanol (7 mL) was heated at 60°C for 3 h. The resulting clear solution was diluted into methylene chloride, washed with one-half saturated aqueous sodium carbonate, brine, dried (Na₂SO₄) and concentrated *in vacuo* to a foam, 0.42 g. Flash chromatography (10% methanol/dichloromethane) afforded a colorless solid, 0.22 g; mp. 90-93°C. ¹H NMR (CDCl₃) δ 7.95 (s, 1H), 7.40 (d, 1H), 7.10 (d, 2H), 6.78 (d, 2H), 6.42 (d, 1H), 4.54 (dd, 1H), 4.36 (s, 2H), 4.00 (t, 2H), 3.63 (s, 3H), 3.52 (s, 2H), 3.06-2.92 (m, 2H), 2.86 (dd, 1H), 2.68 (dd, 1H).
Step E: (4-(2-(2-(6-Aminopyridin-3-yl)-2(R)-hydroxyethylamino)ethoxy)phenyl)acetic acid. To a stirred solution of the product of Example One, Step D, methyl (4-(2-(2-(6-aminopyridin-3-yl)-2(R)-hydroxyethylamino)ethoxy)phenyl)acetic acid (0.11 g, 0.03 mmol), in methanol (6 mL) were added water (1.5 mL) and potassium hydroxide (0.07 g, 1.3 mmol). The resulting solution was stirred at room temperature for 4 h and concentrated *in vacuo.* The resulting mixture was dissolved in water (2 mL) and the pH adjusted to 5.5 with 1N aqueous hydrochloric acid. The precipitate was filtered and dried at 80°C *in vacuo* to afford a colorless solid, 0.10 g; m.p. 233-235°C. ¹H NMR (DMSO-d6) δ 7.81 (s, 1H), 7.33 (d, 1H), 7.13 (d, 2H), 6.86 (d, 2H), 6.40 (d, 1H), 6.00 (brs, 2H), 4.78 (dd, 1H), 4.23 (t, 2H), 3.46 (s, 2H), 3.33 (t, 2H), 3.09 (t, 2H). 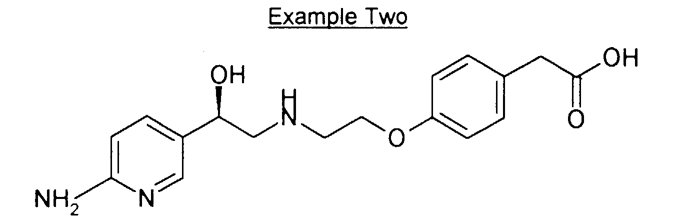
(4-(2-(2-(6-Aminopyridin-3-yl)-2-(R)-hydroxyethylammonium)-ethoxy)-phenyl)-acetate. A mechanically stirred slurry of the title compound of Preparation Five (50.0gm, 0.2806mol, 1.0 eq) and the title compound of Preparation Nine (99.4gm, 0.477mol, 1.7eq) in 5:1 (vol/vol)::Toluene:DMSO (375mL) was heated on a steam bath. The slurry became homogenous at about 70°C, and the temperature was maintained at 90°C-95°C for 3 to 16 hrs. The solution was cooled to 10°C-15°C. This resulted in the formation of a precipitate. Di-*t*-butyldicarbonate (129mL, 0.561 mol, 2.0 eq) was added dropwise over a one hour period. The resulting homogenous solution was stirred at room temperature overnight. The solution was poured into a mixture of ethyl acetate (1L) and water (850 mL). After stirring for 10 min, the phases were allowed to separate, at which time a heavy red oil fell out into the aqueous layer. The aqueous layer, with oil, was removed. The organic layer was washed with water (500mL) and concentrated to an amber oil. This amber oil was taken up in 6N HCI (300mL) and heated on the steam bath overnight. The solution was cooled to room temperature, and the solids which precipitated were filtered. (These solids are the amino acid of the excess side chain which was used in the coupling with the epoxide.) The acidic solution containing the title compound was concentrated under vacuum to a semi-solid. The semi-solid was treated with water and then reconcentrated (twice) to remove excess HCI. The solid was dissolved in water and brought to pH 7 with potassium hydroxide. The solid which precipitated was filtered and washed first with water and then with THF. The solids were dried on the filter funnel to a weight of 22.5 gm. The crude solid was redissolved in 30 volumes of 90 °C water and treated with decolorizing carbon. After filtration to remove the carbon, the filtrate was cooled and concentrated by evaporation of some of the water. The precipitate which formed was filtered to provide 9.5 gm of the title compound. NMR (400MHz, DMSO-*d*₆ + D₂O): d = 7.79 (d, 1H, J=1.87), 7.34-7.32 (m, 1H), 7.11 (d, 2H, J=8.51), 6.79 (d, 2H, J=8.51), 6.41 (d, 1H, J=8.51), 4.54-4.51 (m, 1H), 4.01-3.99 (m, 2H), 3.35 (s, 2H), 2.97-2.94 (m, 2H), 2.79-2.69 (m, 2H). MS(APCI) *m/z* 332.2 (MH⁺), 314.2, 159.1, 156.9.

### Example Three

*In vivo* study for combination therapy with the compound of Formula I and an anorectic agent. Human patients are given an oral dose of 0.1 mg/kg to 10 mg/kg of an anorectic agent in vehicle (anorectic agent treated), administered one to three times per day, together with or followed by 0.1 to 10 mg/kg of the compound of Formula I, administered orally one to three times per day. The first treatment is given on day 0, and the patients are treated daily for a six month period. A set of control patients are untreated (e.g., placebo) for comparison. Body weight data are collected each week. Statistical significance of body weight decreases is determined by performing a two-factor (group and day) analysis of variance (ANOVA) with repeated measures followed by a post hoc Least Squares Difference (LSD) test.

Determination of plasma insulin and glucose levels. Blood is collected into heparinized capillary tubes on the day before the first treatment and daily for the first week. Thereafter, patients should individually monitor their own blood glucose daily (using commercially available kits). Blood is collected for laboratory analysis weekly at the same time that body weight measurements are taken. Blood samples are analyzed (e.g., at a registered hospital or other GLP laboratory) for glucose and insulin levels.

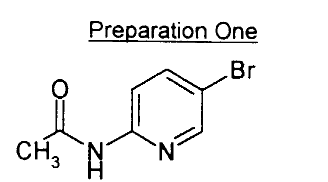

N-(5-Bromo-pyridin-2-yl)-acetamide. A solution of 2-amino-5-bromopyridine (25.0 g, 144 mmol) in acetic acid (50 ml) and acetic anhydride (25.0 g) was heated at reflux for two hours. The reaction mixture was then cooled and poured into water (750 ml) with stirring. After one hour, the solution was adjusted to pH 10 with 50% sodium hydroxide and the precipitate was filtered, washed with water and dried to give 26.5 g (85%) of the title product as a white flaky solid. mp 175-176 °C. ¹H NMR (CDCl₃): δ = 8.29 (d, 1 H); 8.12 (d, 1 H); 7.96 (br, 1 H); 7.78 (d of d, 1 H); 2.19 (s, 3 H). MS (El): *m/z* = 214, 216 (M⁺, Br isotopes).

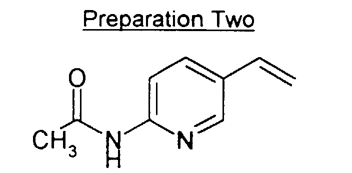

N-(5-Vinyl-pyridin-2-yl)-acetamide. A solution of of N-(5-bromo-pyridin-2-yl)-acetamide (prepared as described in Preparation One, 4.30 g, 20 mmol) in acetonitrile (15 ml) and triethylamine (5.04 ml) was treated with palladium acetate (45 mg, 0.2 mmol) and tri-o-tolylphosphine (203 mg, 0.66 mmol). The mixture was placed in a pressure reactor under 50 psig of ethylene pressure and heated at 85 °C for 66 hours. The reaction mixture was cooled, vented, and partitioned between phosphate buffer (0.1 M, pH 6.6) and ethyl acetate. The aqueous phase was extracted with ethyl acetate twice more. The combined ethyl acetate extracts were washed with additional phosphate buffer, brine and dried over sodium sulfate. The extracts were filtered and evaporated to afford 2.06 g (63%) of the title product as a flaky crystalline residue. Recrystallization from ethyl acetate/cyclohexane gave colorless flakes. mp 120 - 121 °C 1H NMR (CDCl₃): δ = 8.55 (br, 1 H); 8.24 (d, 1 H); 8.15 (d, 1 H); 7.76 (d of d, 1 H); 6.64 (d of d, 1 H); 5.73 (d, 1 H); 5.28 (d, 1 H); 2.19 (s, 3 H). MS (Cl): *m/z =* 163 (M+H⁺).

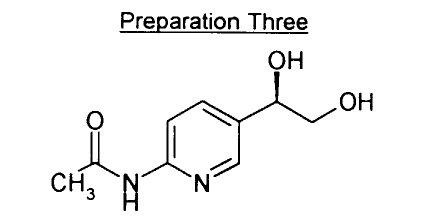

(R)-N-(5-(1,2-Dihydroxy-ethyl)-pyridin-2-yl)-acetamide. A suspension of AD-Mix-B® (56.33 g) in water (200 ml) and t-butanol (200 ml) was cooled to 5 °C and N-(5-vinyl-pyridin-2-yl)-acetamide (prepared as described in Preparation Two, 6.52 g, 40.2 mmol) was added followed by 2-propanol (400 ml). The mixture was stirred at 5 °C for 12 hours and then at 20 °C for 12 hours. The reaction mixture was then treated with sodium sulfite (60.4 g), stirred for 30 minutes and then diluted with 500 ml of 2-propanol and stirred for an additional one hour. The mixture was filtered and the alcoholic phase was separated and evaporated to dryness. The residue was slurried in 500 ml of 2-propanol and evaporated again. The residue was dried to afford 6.35 g (80%) of the title product as colorless crystals. The crystals were recrystallized by dissolving in hot glacial acetic acid, diluting 7-fold with 2-propanol, cooling and seeding to give the title product as crystals. mp 184-185 °C. ¹H NMR (dmso-d₆): δ = 8.22 (d, 1 H); 7.99 (d, 1 H); 7.68 (d of d, 1 H); 4.52 (t, 1 H); 3.44 (m, 2 H); 2.07 (s, 3 H). MS (Cl): *m/z =* 197 (M+H⁺). Optical Rotation: -4.52 ° (c = 0.05, acetic acid). Analysis: Calculated for C₉H₁₂N₂O₃: C, 55.09%; H,6.17%; N, 14.28%. Found: C, 55.43%; H, 5.97%: N, 13.96%.

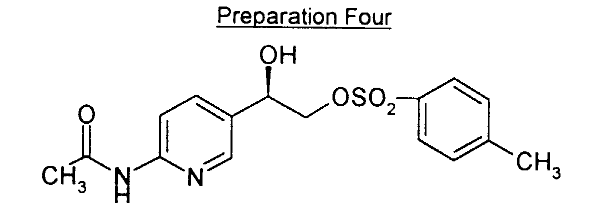

(R)-Toluene-4-sulfonic acid 2-(6-acetylamino-pyridin-3-yl)-2-hydroxy-ethyl ester. A slurry of (R)-N-(5-(1,2-dihydroxy-ethyl)-pyridin-2-yl)-acetamide (prepared as described in Preparation Three, 71.2 g, 362 mmol) in anhydrous pyridine (362 ml) was cooled to 5 °C and treated with p-toluenesulfonyl chloride (69.18 g, 362 mmol) in one portion. The reaction mixture was stirred at 5 °C for 20 minutes, then the cooling bath was removed and the mixture was stirred at ambient temperature for two hours. The mixture was then concentrated, dissolved in 30 ml of methanol, concentrated and dissolved in toluene (300 ml) and concentrated again. The residue was treated again with methanol and toluene, then the residue was dissolved in ethyl acetate and washed sequentially with half-saturated brine, brine and dried over sodium sulfate. The filtrate was evaporated to afford 102.2 g (80%) of the title product as light buff crystals. Recrystallization from ethanol-cyclohexane afforded the title product as colorless crystals. mp 124-126 °C. ¹H NMR (dmso-d₆): δ = 10.5 (br, 1 H); 8.21 (d, 1 H); 7.94 (d, 1 H); 7.68 (d, 2 H); 7.51 (d of d, 1 H); 7.41 (d, 1 H); 5.87 (d, 1 H); 4.76 (d of d, 1 H); 4.05 (d, 2 H); 2.41 (s, 3 H); 2.10 (s, 3 H). MS (Cl): *m/z* 351 (M+H⁺). Optical Rotation: -36.181 ° (c = 1.19, acetone). Analysis: Calculated for C₁₆H₁₈N₂O₅S: C, 54.85%; H, 5.18%; N, 7.99%. Found: C, 54.91%; H, 5.34%; N, 8.06%.

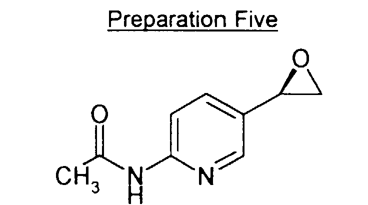

(R)-N-(5-Oxiranyl-pyridin-2-yl)-acetamide. A solution of (R)-toluene-4-sulfonic acid 2-(6-acetylamino-pyridin-3-yl)-2-hydroxy-ethyl ester (prepared as described in Preparation Four, 200 g, 0.57 mol) in THF (2.4 L) was cooled to -15°C and potassium t-butoxide (542 ml, 0.542 mol, 1M in THF) was added slowly at -15°C to -10°C over a two hour period. Stirring was continued at -15°C for an additional 40 minutes. The reaction mixture was filtered with the aid of Celite®. The filtration was done through cloth precoated with Celite®. The filter cake was washed with tetrahydrofuran. The filtrate was concentrated under vacuum to afford 300 ml of an oil. The oil was diluted with 1.2 liters of hexanes which resulted in the formation of a solid. The suspension was stirred at room temperature for one hour to granulate the solid. The suspension was filtered and the filtrate was washed with hexanes to afford 80.0 g (78.8%) of the title product as a solid. mp 96-98°C. ¹H NMR (CDCl₃): δ = 8.70 (br, 1 H); 8.21 (m, 2 H); 7.57 (d of d, 1 H); 3.86 (m, 1 H); 3.17 (m, 1 H); 2.83 (m, 1 H); 2.19 (s, 3 H). MS (Cl): *m/z* = 179 (M+H⁺).

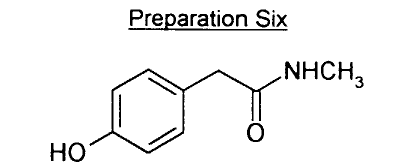

N-Methyl 4-hydroxyphenylacetamide. Monomethylamine (22.43 kg, 722.15 mol, 6 eq.) was added over a 7-hour period to a solution of methyl-4-hydroxyphenylacetate (20.0 kg, 120.35 mol, 1.0 eq.) in methanol (31.7 gal, 120 L) and stirred overnight at room temperature. Methanol was then displaced under vacuum with ethyl acetate. The resulting slurry (about 20 gal, 75.7 L) was stirred at +10°C for 1 hour, then filtered and dried under vacuum at 45°C to yield of the title compound(18.68 kg, 94% of theory).
mp 124-125°C. NMR (300 MHz, *d*₆-DMSO): δ = 9.26 (s, 1H), 8.00-7.65 (br s, 1H), 7.21-6.90 (m, 2H), 6.86-6.55 (m, 2H), 3.26 (s, 2H), 2.75-2.45 (m, 3H).

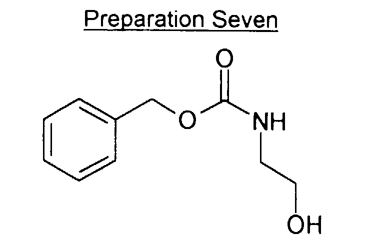

N-Benzyloxycarbonyl-2-aminoethanol. Benzylchloroformate (44.95 kg, 263.5 mol, 1.0 eq.) was added over a 2 hour period at room temperature to a solution of ethanolamine (16.1 kg, 263.5 mol, 1.0 eq.) in water (34 gal, 128.7 L). After stirring for 30 minutes, this was added to a cold (5-10°C) solution of NaHCO₃ (33.2 kg, 395.25 mol, 1.5 eq) in H₂O (330 L) over a 30 min period and then allowed to stir at room temperature overnight. Ethyl acetate (22 gal, 83.3 L) was added, the layers separated, and the aqueous layer extracted again with ethyl acetate (22 gal., 83.3 L). The combined organic extracts were concentrated under vacuum to a volume of 10 gal (37.9 L), and the remainder displaced with isopropyl ether. The resulting slurry was stirred and cooled to 10°C for 2 hours, then filtered. The solids were washed with isopropyl ether and vacuum dried to give the title compound (39.1 kg, 71.1%). mp 61-63°C. NMR (300 MHz, *d*₆-DMSO): δ = 7.50-7.37 (m, 5H), 7.37-7.16 (m, 1H), 5.05 (s, 2H), 4.70-4.63 (m, 1H), 3.46-3.37 (m, 2H), 3.13-3.03 (m, 2H).

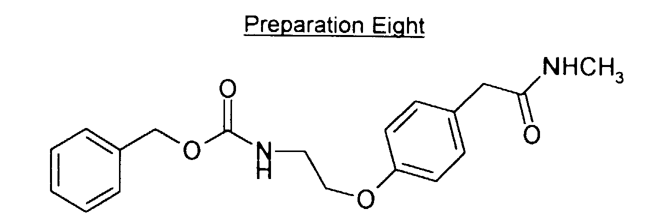

Methyl 4-(2-(N-benzyloxycarbonylamino)ethoxy)phenylacetamide. The title compound of Preparation Six (18.68 kg, 113.14 mol, 1.0 eq.) and the title compound of Preparation Seven (33.13 kg, 169.75 mol, 1.5 eq.) were dissolved in THF (40 gal, 151.4 L). Triphenylphosphine (44.5 kg, 169.75 mol, 1.5 eq.) was added and the mixture cooled to -5°C. Diisopropyl azodicarboxylate (34.3 kg, 169.75 mol, 1.5 eq.) was added over an 8 hour period, and the reaction allowed to warm to room temperature overnight. Ethyl acetate (20 gal, 75.7 L) was added to the resulting white slurry, stirring was continued for 6 hours, and the solids filtered off and dried to yield crude title compound. (29.6 kg, 76.5% of theory, mp 131-133°C). The crude product was slurried in ethyl acetate (39.1 gal, 148 L) for 3 hours at 10°C, then filtered, washed with 10°C ethyl acetate (14 gal, 53 L), and vacuum dried to yield the title compound(26.1 kg, 88.2 % recovery, 67.5% overall). mp 134-136°C. NMR (300 MHz, *d*₆-DMSO): δ = 7.98-7.82 (m, 1H), 7.58-7.49 (m, 1H), 7.42-7.28 (m, 5H), 7.20-7.10 (d, 2H), 6.90-6.80 (d, 2H), 5.06 (s, 2H), 4.02-3.93 (m, 2H), 3.47-3.29 (m, 4H), 2.62-2.54 (d, 3H).

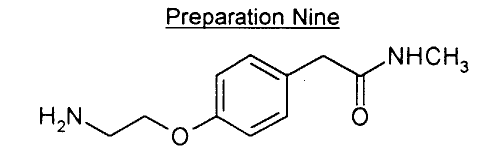

Methyl 4-(2-aminoethoxy)phenylacetamide. The title compound of Preparation Eight (18.4 kg, 53.73 mol) and 1.84 kg 10% palladium on carbon (50% water wet) were suspended in methanol (73 gal, 276.3 L) under nitrogen (N₂), and the reaction vessel pressurized to 50 psig (35.5 x 10³ kg/m²) with hydrogen (H₂) gas. This H₂ pressure was maintained by additional charges of H₂ until there was no further uptake of H₂ (approx. 20 hours) and the reaction was complete by tlc. After purging the vessel with N₂, the mixture was heated to 45°C and filtered at this temperature through Celite®. The solvent was displaced with toluene until a final volume of 8 gal (30.3 L) was achieved. After cooling to 5°C. the resulting solids were filtered off, washed with cold toluene, and vacuum dried to give the title compound (9.95 kg, 88.9% of theory). NMR (300 MHz, d₆-DMSO): δ = 7.99-7.57 (m, 1H), 7.20-7.10 (d, 2H), 6.90-6.80 (d, 2H), 3.93-3.83 (m, 2H), 3.30 (s, 2H), 3.00-2.62 (m, 4H), 2.57 (d, 2H).

While the foregoing specification discloses the principles of the present invention, with examples provided for the purpose of illustration, it will be understood that the practice of the invention encompasses all of the usual variations, adaptations or modifications as come within the scope of the following claims and its equivalents.

## Claims

1. A pharmaceutical composition comprising a compound which modifies eating behavior, a prodrug thereof or a pharmaceutically acceptable salt of said compound or said prodrug; and (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug.

2. A composition of claim 1 wherein said compound which modifies eating behavior is an anorectic agent.

3. A composition of claim 2 further comprising a pharmaceutically acceptable carrier or diluent.

4. A composition of claim 3 wherein said anorectic agent is phenylpropanolamine, ephedrine, pseudoephedrine, phentermine, an NPY antagonist, a CCK-A agonist, a monoamine reuptake inhibitor, a sympathomimetic agent, a serotoninergic agent, a dopamine agonist, a melanocyte-stimulating hormone receptor agonist or mimetic, a cannabinoid receptor antagonist, a melanocyte-stimulating hormone analog, a melanin concentrating hormone antagonist, leptin, a leptin analog or a galanin antagonist.

5. A composition of claim 4 wherein said monoamine reuptake inhibitor is sibutramine.

6. A composition of claim 4 wherein said serotoninergic agent is dexfenfluramine or fenfluramine.

7. A composition of claim 4 wherein said anorectic agent is leptin or a leptin analog.

8. A composition of claim 4 wherein said dopamine agonist is bromocriptine.

9. A composition of claim 4 wherein said anorectic agent is a CCK-A agonist.

10. A composition of claim 4 wherein said anorectic agent is a galanin antagonist.

11. A method of treating obesity in an animal comprising administering to said animal a therapeutically effective amount of a compound which modifies eating behavior, a prodrug thereof or a pharmaceutically acceptable salt of said compound or said prodrug; and (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug.

12. A method of claim 11 wherein said compound which modifies eating behavior is an anorectic agent.

13. A method of claim 12 wherein said anorectic agent is phenylpropanolamine, ephedrine, pseudoephedrine, phentermine, an NPY antagonist, a CCK-A agonist, a monoamine reuptake inhibitor, a sympathomimetic agent, a serotoninergic agent, a dopamine agonist, a melanocyte-stimulating hormone receptor agonist or mimetic, a cannabinoid receptor antagonist, a melanocyte- stimulating hormone analog, a melanin concentrating hormone antagonist, leptin, a leptin analog or a galanin antagonist.

14. A method of claim 13 wherein said monamine reuptake inhibitor is sibutramine.

15. A method of claim 13 wherein said serotoninergic agent is fenfluramine or dexfenfluramine.

16. A method of claim 13 wherein said anorectic agent is leptin or a leptin analog.

17. A method of claim 13 wherein said dopamine agonist is bromocriptine.

18. A method of claim 13 wherein said anorectic agent is a CCK-A agonist.

19. A method of claim 13 wherein said anorectic agent is a galanin antagonist.

20. A method of treating diabetes in an animal comprising administering to said animal a therapeutically effective amount of a compound which modifies eating behavior, a prodrug thereof or a pharmaceutically acceptable salt of said compound or said prodrug; and (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug.

21. A method of claim 20 wherein said compound which modifies eating behavior is an anorectic agent.

22. A method of claim 21 wherein said anorectic agent is phenylpropanolamine, ephedrine, pseudoephedrine, phentermine, an NPY antagonist, a CCK-A agonist, a monoamine reuptake inhibitor, a sympathomimetic agent, a serotoninergic agent, a dopamine agonist, a melanocyte-stimulating hormone receptor agonist or mimetic, a cannabinoid receptor antagonist, a melanocyte- stimulating hormone analog, a melanin concentrating hormone antagonist, leptin, a leptin analog or a galanin antagonist.

23. A method of claim 22 wherein said monamine reuptake inhibitor is sibutramine.

24. A method of claim 22 wherein said serotoninergic agent is fenfluramine or dexfenfluramine.

25. A method of claim 22 wherein said anorectic agent is leptin or a leptin analog.

26. A method of claim 22 wherein said dopamine agonist is bromocriptine.

27. A method of claim 22 wherein said anorectic agent is a CCK-A agonist.

28. A method of claim 22 wherein said anorectic agent is a galanin antagonist.

29. A kit comprising:
a. an amount of a compound which modifies eating behavior, a prodrug thereof or a pharmaceutically acceptable salt of said compound or said prodrug in a first unit dosage form;
b. an amount of (4-(2-(2-(6-aminopyrid-3-yl)-2(R)-hydroxyethylamino)ethoxy)phenyl)acetic acid, a prodrug thereof or a pharmaceutically acceptable salt of said (4-(2-(2-(6-aminopyrid-3-yl)-2(R)-hydroxyethylamino)ethoxy)phenyl)acetic acid or said prodrug in a second unit dosage form; and
c. a container.

30. A kit of claim 29 wherein said first unit dosage form further comprises a pharmaceutically acceptable carrier or diluent.

31. A kit of claim 30 wherein said compound which modifies eating behavior is an anorectic agent.

32. A kit of claim 29 wherein said second unit dosage form further comprises a pharmaceutically acceptable carrier or diluent.

33. A kit of claim 32 wherein said compound which modifies eating behavior is an anorectic agent.

34. A kit of claim 29 wherein said first and second unit dosage forms each further comprise a pharmaceutically acceptable carrier or diluent.

35. A kit of claim 34 wherein said compound which modifies eating behavior is an anorectic agent.

36. A composition of claim 3 wherein said anorectic agent is a bombesin agonist, dehydroepiandrosterone or analog thereof, glucocorticoid receptor agonist or analog thereof, an orexin receptor antagonist, a urocortin binding protein antagonist or a glucagon-like peptide-1 (insulinotropin) agonist.

37. A method of claim 12 wherein said anorectic agent is a bombesin agonist, dehydroepiandrosterone or analog thereof, glucocorticoid receptor agonist or analog thereof, an orexin receptor antagonist, a urocortin binding protein antagonist or a glucagon-like peptide-1 (insulinotropin) agonist.

38. A method of claim 21 wherein said anorectic agent is a bombesin agonist, dehydroepiandrosterone or analog thereof, glucocorticoid receptor agonist or analog thereof, an orexin receptor antagonist, a urocortin binding protein antagonist or a glucagon-like peptide-1 (insulinotropin) agonist.
